# EUROPÄISCHE PATENTSCHRIFT

(11) **EP 2 474 301 B1**
(45) Veröffentlichungstag und Bekanntmachung des Hinweises auf die Patenterteilung: **16.04.2014**
(21) Anmeldenummer: 11193578.9
(22) Anmeldetag: 14.12.2011
(51) Int. Cl.: A61K 8/37, A61Q 13/00, C11D 3/50, C11B 9/00

(54) **Riechstoffmischungen enthaltend Cyclopent-2-Enyl-Essigsäureethylester**
Perfume mixtures containing Cyclopent-2-Enyl-ethyl acetate
Mélanges de parfum comprenant du cyclopent-2-ényl-éthylester d'acide acétique

(43) Veröffentlichungstag der Anmeldung: 11.07.2012
(73) Patentinhaber: Symrise AG, 37603 Holzminden (DE)
(72) Erfinder: Hölscher, Bernd, 37620 Halle (DE); Chmelnyk, Annabel, 37603 Holzminden (DE)
(74) Vertreter: Eisenführ Speiser

(56) Entgegenhaltungen:
- GB-A- 391 579
- US-A- 4 584 127
- US-A- 5 962 706
- US-A1- 2009 036 692

## Beschreibung

Die vorliegende Erfindung betrifft Riechstoffmischungen, vorzugsweise Parfümöle, umfassend eine

Verbindung der Formel (I) (Cyclopent-2-enyl-essigsäureethylester) 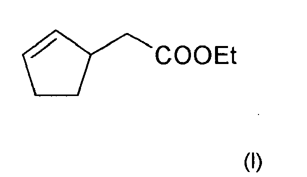 sowie zusätzlich einen oder mehrere weitere Riechstoffe.

Ferner betrifft die vorliegende Erfindung Verfahren zur Herstellung von erfindungsgemäßen Riechstoffmischungen, insbesondere Parfümölen, parfümierte Produkte enthaltend eine erfindungsgemäße Riechstoffmischung, Verfahren zur Herstellung von erfindungsgemäßen parfümierten Produkten sowie die Verwendung der Verbindung der Formel (I) zum Verstärken der natürlichen Frische und/oder Ausstrahlung und/oder zum Maskieren oder Vermindern fettiger, technischer und/oder metallischer Noten eines oder mehrerer von der Verbindung der Formel (I) verschiedener Riechstoffe.

Die Verbindung der Formel (I) ist dem Fachmann bekannt, eine Geruchsbeschreibung der Verbindung der Formel (I) findet sich in dem Fachbuch "S. Arctander, Perfume and Flavor Materials, Vol. I und II, Montclair, N. J., 1969, Selbstverlag" unter der Nummer 1205. Die Verbindung mit der Formel (I) wird dort als stark, frisch-fruchtig mit einer Note von einer überreifen Ananas beschrieben. Dem Lehrbuch kann ebenfalls entnommen werden, dass die Verbindung mit der Formel (I) in einigen Aromenmischungen eingesetzt wird. In der Parfümerie fand die Verbindung mit der Formel (I) bisher keine nennenswerte Anwendung. Die Nennung von bestimmten Mengenverhältnissen oder bestimmten geruchlichen Effekten bei Kombination mit Riechstoffen der Bestandteile (b) oder (c) ist bisher nicht bekannt.

Die Verbindung der Formel (I) (Cyclopent-2-enyl-essigsäureethylester; CAS Nr.: 15848-49-4) kann z. B. durch Verseifung und anschließende Decarboxylierung von 2-Cyclopenten-1-Malonsäurediethylester erhalten werden, der wiederum nach der Synthesevorschrift von Moffett et al. (Organic Syntheses, Coll. Vol. 4, p.291 (1963); Vol. 32, p.52 (1952)) hergestellt werden kann.

Blumenriechstoffe spielen in der Parfümerie eine wichtige Rolle. Es besteht ein ständiger Bedarf, bestimmte geruchliche Aspekte eines Riechstoffes oder einer Riechstoffmischung zu betonen (hervorzuheben), im Falle von Blumenriechstoffen gilt dies insbesondere für deren natürliche Frische und Ausstrahlung. Ebenso besteht ein ständiger Bedarf, bestimmte geruchliche Aspekte eines Riechstoffes oder einer Riechstoffmischung zu maskieren oder zu vermindern, im Falle von Blumenriechstoffen gilt dies insbesondere für fettige und technische Noten.

Die Aufgabe der vorliegenden Erfindung war es, bestimmte geruchliche Aspekte bestimmter Riechstoffe oder Riechstoffmischungen zu betonen bzw. hervorzuheben und/oder bestimmte geruchliche Aspekte eines Riechstoffs oder einer Riechstoffmischung zu maskieren oder zu vermindern, insbesondere fettige und technische Noten.

Gelöst wird diese Aufgabe durch eine Riechstoffmischung, vorzugsweise durch ein Parfümöl, umfassend die Bestandteile
(a) Verbindung der Formel (I) 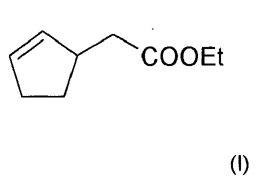 sowie zusätzlich:
(b) einen oder mehrere Riechstoffe, vorzugsweise mit einer blumigen Geruchsnote, aus der Gruppe bestehend aus Alkoholen und Aldehyden mit einer Molmasse von 210 g/mol oder weniger
   und/oder
(c) einen oder mehrere Riechstoffe aus der Gruppe bestehend aus Ketonen, Ethern und Estern mit einer Molmasse im Bereich von 190 g/mol bis 250 g/mol.

Erfindungsgemäße Riechstoffmischungen sind regelmäßig bei 25°C und 1013hPa flüssig und sind üblicherweise homogene Lösungen.

Überraschenderweise bewirkt in einer erfindungsgemäßen Riechstoffmischung die Verbindung der Formel (I), dass bestimmte geruchliche Aspekte des oder der Riechstoffe der Komponente (b) und/oder des oder der Riechstoffe der Komponente (c) betont bzw. hervorgehoben und/oder maskiert bzw. vermindert werden. Insbesondere fettige und technische Noten der Riechstoffe der Komponenten (b) und/oder (c) werden durch die Verbindung der Formel (I) effektiv maskiert bzw. vermindert.

Der Fachmann wird in einer erfindungsgemäßen Riechstoffmischung (beispielsweise einem Parfümöl) den Anteil an Komponente (a), d. h. den Anteil an der Verbindung der Formel (I), so wählen, dass der von ihm gewünschte Effekt des Betonens (Hervorhebens) und/oder Maskierens bzw. Verminderns einer Geruchsnote erreicht wird, wobei er Sorge tragen wird, keine zu große Menge der Komponente (a) einzusetzen, welche den sensorischen Gesamteindruck einer Riechstoffmischung dominieren könnte und andererseits nicht lediglich eine so geringe Menge der Komponente (a) vorzusehen, dass eine Betonung bzw. Maskierung/Verminderung geruchlicher Aspekte von Riechstoffen der Komponente (b) bzw. (c) nicht bzw. kaum noch zu spüren ist. Zu bevorzugten Konzentrationsverhältnissen vergleiche die nachfolgenden Ausführungen und die beigefügten Beispiele.

Überraschenderweise hat sich gezeigt, dass die Verbindung der Formel (I) über ihre primären sensorischen Eigenschaften hinaus zusätzliche positive Sekundäreigenschaften, wie z.B. eine hohe Stabilität unter bestimmten Anwendungsbedingungen (in alkalischen Medien (Waschpulver, Wäscheweich, Seife, Shampoo etc.), eine hohe Ausgiebigkeit, ein gutes Haftungsvermögen, eine hohe Substantivität aufweist. Diese Eigenschaft ist für einen Ester mit einem Molgewicht von 154 g/mol.

### Bestandteil (b) einer erfindungsgemäßen Riechstoffmischung

Bestandteil (b) der erfindungsgemäßen Riechstoffmischung umfasst einen oder mehrere Riechstoffe aus der Gruppe bestehend aus Alkoholen und Aldehyden mit einer Molmasse von 210 g/mol weniger. Bevorzugt weisen diese Riechstoffe eine blumige Geruchsnote auf. Solche Riechstoffe sind dem Fachmann bekannt; Alkohole und Aldehyde (auch solche mit einer blumigen Geruchsnote) stellen eine sehr wichtige Riechstoffgruppe in der Parfümerie dar.

Bevorzugt sind erfindungsgemäße Riechstoffmischungen, vorzugsweise Parfümöle, wobei der Bestandteil (b) zwei, drei, vier, fünf oder mehr verschiedene Riechstoffe enthält.

Vorzugsweise ist in einer erfindungsgemäßen Riechstoffmischung das Massenverhältnis der Gesamtmenge an Riechstoffen des Bestandteils (b) zur Verbindung der Formel (I) größer oder gleich 99 : 1, bevorzugt größer oder gleich 99,9 : 0,1, besonders bevorzugt größer oder gleich 99,999 : 0,001 besonders bevorzugt.

In eigenen Untersuchungen hat sich gezeigt, dass diese Massenverhältnisse besonders vorteilhaft sind. Bei diesen Massenverhältnissen ist der Eigengeruch der Verbindung der Formel (I) regelmäßig nicht mehr oder kaum noch wahrnehmbar, jedoch bewirkt die Anwesenheit der Verbindung der Formel (I) einen positiven Einfluss auf die Gesamtnote der erfindungsgemäßen Riechstoffmischung. Besonders überraschend ist, dass die Verbindung der Formel (I) auch in geringen Konzentrationen eine Auswirkung auf die Frische und Ausstrahlung der Riechstoffmischung hat, ohne in relevantem Maße einen fruchtigen Geruch zu bewirken oder zu betonen.

Besonders bevorzugt sind erfindungsgemäße Riechstoffmischungen, vorzugsweise erfindungsgemäße Parfümöle, wobei der oder die Riechstoffe des Bestandteils (b) ausgewählt sind aus der Gruppe bestehend aus 2-Methyl-3-(4-tert-butylphenyl)propanal, 4-(4-Hydroxy-4-methylpentyl)-3-cyclohexencarboxaldehyd, 2-Methyl-4-(2,2,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-l-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

Besonders bevorzugt sind erfindungsgemäße Riechstoffe, vorzugsweise erfindungsgemäße Parfümöle, wobei der oder die Riechstoffe des Bestandteils (b) ausgewählt sind aus der Gruppe bestehend aus 1-(4-Isopropyl-cyclohexyl)-ethanol (Mugetanol), (E)-3,7-Dimethyl-octa-2,6-dien-1-ol (Geraniol), 3,7-Dimethyl-oct-6-en-1-ol (Citronellol), (4-Isopropyl-cyclohexyl)-methanol (Mayol), 2-Methyl-6-methylenoct-7-en-2-ol (Myrcenol) (2-Methyl-6-methylenoct-7-en-2-ol) und 2,6-Dimethyl-oct-7-en-2-ol (Dihydromyrcenol).

Bevorzugt sind erfindungsgemäße Riechstoffmischungen, vorzugsweise erfindungsgemäße Parfümöle, wobei der, mehrere oder sämtliche Riechstoffe des Bestandteils (b) jeweils eine Molmasse im Bereich von 140 bis 170 g pro mol aufweisen.

Ganz besonders bevorzugt sind erfindungsgemäße Riechstoffmischungen, vorzugsweise erfindungsgemäße Parfümöle, in deren der Bestandteil (b) ein Alkohol ist.

Überraschenderweise werden die sensorischen Eigenschaften von Riechstoffen der Komponente (b) durch Kombination mit einer Menge der Verbindung der Formel (I) positiv beeinflusst. Im Einzelfall wird der sensorische Eindruck in Richtung natürlicher, frischer, blumiger, mehr Ausstrahlung, weniger technisch, weniger fettig und/oder weniger metallisch verschoben, wobei im Einzelfall selbstverständlich auch weitere sensorische Einflüsse zu beobachten waren. Detaillierte Geruchsbeschreibungen finden sich in den beigefügten Beispielen.

### Bestandteil (c) einer erfindungsgemäßen Riechstoffmischung

Bevorzugt sind erfindungsgemäße Riechstoffmischungen, vorzugsweise Parfümöle, wobei der Bestandteil (c) zwei, drei, vier, fünf oder mehr verschiedene Riechstoffe enthält.

Die Riechstoffe des Bestandteils (c) fungieren regelmäßig als Fondnoten einer erfindungsgemäßen Riechstoffmischung bzw. eines erfindungsgemäßen Parfümöls.

Vorzugsweise handelt es sich bei den Riechstoffen des Bestandteils (c) um Ketone, Ether und/oder Ester mit einer Molmasse im Bereich von 196 g/mol bis 250 g/mol.

Verbindungen mit einer Molmasse zwischen 190 und 210 g/mol, die sowohl zu der Gruppe der Aldehyde und/oder Alkohole als auch zu der Gruppe der Ketone, Ester und/oder Ether gehören, werden sowohl dem Bestandteil (b) als auch dem Bestandteil (c) zugeordnet.

Besonders bevorzugt sind erfindungsgemäße Riechstoffmischungen, vorzugsweise erfindungsgemäße Parfümöle, in denen das Massenverhältnis der Gesamtmenge an Riechstoffen des Bestandteils (c) zur Verbindung der Formel (I) größer oder gleich 99 : 1 ist, bevorzugt größer oder gleich 99,9 : 0,1, besonders bevorzugt größer oder gleich 99,999 : 0,001 ist.

In eigenen Untersuchungen hat sich gezeigt, dass diese Massenverhältnisse besonders vorteilhaft sind. Bei diesen Massenverhältnissen ist der Eigengeruch der Verbindung der Formel (I) regelmäßig nicht mehr bzw. kaum noch wahrnehmbar, jedoch bewirkt die Anwesenheit der Verbindung der Formel (I) einen positiven Einfluss auf die Gesamtnote der erfindungsgemäßen Riechstoffmischung. Besonders überraschend ist, dass die Verbindung der Formel (I) auch in geringen Konzentrationen eine Auswirkung auf die Frische und Ausstrahlung der Riechstoffmischung hat, ohne in relevantem Maße einen fruchtigen Geruch zu bewirken oder zu betonen.

Beispiele für Riechstoffe mit einem Molgewicht im Bereich von 190 g/mol bis 250 g/mol, die Teil des Bestandteils (c) sein können, sind dem Fachmann bekannt und beispielsweise zu finden in S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Besonders bevorzugt sind erfindungsgemäße Riechstoffmischungen, vorzugsweise erfindungsgemäße Parfümöle, wobei der oder die Riechstoffe des Bestandteils (c) ausgewählt sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, Oxacyclohexa-decan-2-on, 15-Hydroxy-Pentadecanonsäurelacton, 5-Cyclohexadecen-1-on, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 8-Cyclohexadecen-1-on, 3a,6,6,9a-Tetramethyl dodecahydronaphtho[2,1-b]furan, alpha-Iron, beta-Iron, alpha-n-Methylionon, beta-n-Methylionon, alpha-Isomethylionon, beta-Isomethylionon und Allylionon.

Besonders bevorzugte erfindungsgemäße Riechstoffmischungen sind solche, in denen Bestandteil (c) Methyldihydrojasmonat enthält oder aus Methyldihydrojasmonat besteht.

Besonders bevorzugte erfindungsgemäße Riechstoffmischungen, vorzugsweise erfindungsgemäße Parfümöle, bei denen Bestandteil (c) Methyldihydrojasmonat enthält oder aus Methyldihydrojasmonat besteht, sind solche, bei denen der Gehalt an cis-Methyldihydrojasmonat mehr als 30 Gew.-%, weiter bevorzugt mehr als 60 Gew.-%, besonders bevorzugt mehr als 75 Gew.-% und ganz besonders bevorzugt mehr als 90 Gew.-% beträgt, jeweils bezogen auf die Gesamtmasse an cis- und trans-Methyldihydrojasmonat.

In eigenen Untersuchungen hat sich gezeigt, dass erfindungsgemäße Riechstoffmischungen, insbesondere Parfümöle, in denen Bestandteil (c) Methyldihydrojasmonat enthält oder aus Methyldihydrojasmonat besteht, aufgrund der Anwesenheit der Verbindung der Formel (I) einen besonders bevorzugten Geruchseindruck vermitteln. Die Verbindung der Formel (I) bewirkt eine Harmonisierung und Abrundung des individuellen Geruches von Methyldihydrojasmonat und bewirkt im Zusammenspiel einen natürlichen floralen Geruch von weißen Blüten. Die Jasminnoten des Methyldihydrojasmonat werden in einzigartiger Weise betont, so dass die gesamte Komposition wertvoller erscheint.

Überraschenderweise werden die sensorischen Eigenschaften von Riechstoffen der Komponente (c) durch Kombination mit einer Menge der Verbindung der Formel (I) positiv beeinflusst. Im Einzelfall wird der sensorische Eindruck in Richtung natürlicher, frischer, blumiger, mehr Ausstrahlung, weniger technisch, weniger fettig und/oder weniger metallisch verschoben, wobei im Einzelfall selbstverständlich auch weitere sensorische Einflüsse zu beobachten waren. Detaillierte Geruchsbeschreibungen finden sich in den beigefügten Beispielen.

In erfindungsgemäßen Riechstoffmischungen wird die Verbindung der Formel (I) selbstverständlich mindestens in einer solchen Menge eingesetzt, dass eine sensorische Wirkung erzielt wird. Eine sensorische Wirkung wird durch die Anwesenheit der Verbindung der Formel (I) dann erzielt, wenn eine Vergleichs-Riechstoffmischung, die bei ansonsten identischer Zusammensetzung keine Verbindung der Formel (I) enthält, sensorisch anders bewertet wird als die erfindungsgemäße Riechstoffmischung.

Vorzugsweise wird die Verbindung der Formel (I) in einer erfindungsgemäßen Riechstoffmischung in einer solchen Konzentration eingesetzt, dass der sensorische Eindruck der erfindungsgemäßen Riechstoffmischung natürlicher, frischer, blumiger, mehr Ausstrahlung besitzend, weniger technisch, weniger fettig und/oder weniger metallisch ist als der sensorische Eindruck einer Vergleichs-Riechstoffmischung, die bei ansonsten identischer Zusammensetzung keine Verbindung der Formel (I) enthält.

Vorzugsweise ist das Massenverhältnis der Gesamtmenge an Riechstoffen der Bestandteile (b) und (c) zur Verbindung der Formel (I) kleiner oder gleich 99,999 : 0,0001.

Erfindungsgemäße Riechstoffmischungen, insbesondere erfindungsgemäße Parfümöle, können in flüssiger Form, unverdünnt oder mit einem Lösungsmittel verdünnt zur Parfümierung oder Aromatisierung eingesetzt werden. Geeignete Lösungsmittel hierfür sind insbesondere Ethanol, Glycerin, 1,2-Propylenglycol, 1,2-Butylenglycol, Dipropylenglycol, Diethylphthalat, Triethylcitrat, Isopropylmyristat und Triacetin.

Alkohole und Aldehyde mit einer Molmasse von 210 g/mol oder weniger sowie Ketone, Ether und Ester mit einer Molmasse im Bereich von 190 g/mol bis 250 g/mol werden nicht zu den Bestandteilen (b) bzw. (c) gezählt, sofern es sich um eine Verbindung ausgewählt aus der Gruppe bestehend aus Ethanol, Isopropanol, Glycerin, 1,2-Propylenglycol, 1,2-Butylenglycol, Dipropylenglycol, Diethylphthalat und Triacetin handelt.

Bevorzugt werden erfindungsgemäße Riechstoffmischungen, vorzugsweise erfindungsgemäße Parfümöle, mit weiteren Bestandteilen kombiniert. Bevorzugte weitere Bestandteile sind ausgewählt aus der Gruppe bestehend aus:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie weitere zusätzliche Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten weiteren Riechstoffe, die nicht bereits Teil der Bestandteile (b) sowie (c) einer erfindungsgemäßen Riechstoffmischung bzw. eines erfindungsgemäßen Parfümöls sind, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten. Verbindungen, die unter die Definition der Bestandteile (b) und/oder (c) fallen, werden allerdings unabhängig vom Einsatzzweck diesen Bestandteilen zugeordnet; zu Ausnahmen für bestimmte Lösungsmittel siehe oben.

Des weiteren können erfindungsgemäße Riechstoffmischungen, insbesondere erfindungsgemäße Parfümöle, an einem Trägerstoff adsorbiert sein, der sowohl für eine feine Verteilung der darin enthaltenen Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Riechstoffmischung und Trägerstoff stellt ein beispielhaftes erfindungsgemäßes Produkt dar.

Erfindungsgemäße Riechstoffmischungen, insbesondere erfindungsgemäße Parfümöle können auch mikroverkapselt, sprühgetrocknet, als Einschluss-Komplexe oder als Extrusions-Produkte (d. h. erfindungsgemäße Produkte) vorliegen und in dieser Form z.B. einem zu parfümierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Produkte dar.

Die Mikroverkapselung der erfindungsgemäßen Riechstoffmischungen, vorzugsweise der erfindungsgemäßen Parfümöle, kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die erfindungsgemäße Riechstoffmischung, vorzugsweise ein Parfümöl, enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen der erfindungsgemäßen Riechstoffmischung, vorzugsweise einem erfindungsgemäßen Parfümöl, und Cyclodextrinen oder Harnstoffderivaten in ein geeignetes Lösungsmittel, z.B. Wasser, hergestellt werden. Extrusions-Produkte können durch Verschmelzen einer erfindungsgemäßen Riechstoffmischung, vorzugsweise eines erfindungsgemäßen Parfümöls, mit einem geeigneten wachsartigen Stoff und durch Extrusion mit nachfolgender Erstarrung, ggf. in einem geeigneten Lösungsmittel, z.B. Isopropanol, erhalten werden.

Die Erfindung betrifft zudem ein Verfahren zur Herstellung einer erfindungsgemäßen Riechstoffmischung, vorzugsweise eines erfindungsgemäßen Parfümöls, gekennzeichnet durch folgenden Schritt:
- Mischung des Bestandteils (a) mit Bestandteil (b) und/oder Bestandteil (c). Bevorzugt ist ein erfindungsgemäßes Verfahren, bei dem eine erfindungsgemäße Riechstoffmischung, vorzugsweise ein erfindungsgemäßes Parfümöl, resultiert, in der
- das Massenverhältnis der Gesamtmenge an Riechstoffen des Bestandteils (b) zur Verbindung der Formel (I) größer oder gleich 99 : 1 ist, bevorzugt größer oder gleich 99,9 : 0,1, besonders bevorzugt größer oder gleich 99,999 : 0,001 ist, und/oder
- das Massenverhältnis der Gesamtmenge an Riechstoffen des Bestandteils (c) zur Verbindung der Formel (I) größer oder gleich 99 : 1 ist, bevorzugt größer oder gleich 99,9 : 0,1, besonders bevorzugt größer oder gleich 99,999 : 0,001 ist.

Es hat sich gezeigt, dass die entsprechenden Massenverhältnisse besonders vorteilhaft sind. Auf die obigen Erläuterungen wird verwiesen.

Die Erfindung betrifft zudem ein Verfahren zum Verstärken der natürlichen Frische und/oder Ausstrahlung und/oder zum Maskieren oder Vermindern fettiger, technischer und/oder metallischer Noten eines oder mehrerer von der Verbindung der Formel (I) verschiedener Riechstoffe, insbesondere eines oder mehrerer von der Verbindung der Formel (I) verschiedener Riechstoffe mit einer blumigen Geruchsnote, insbesondere Jasmin, umfassend den folgenden Schritt:
- Vermischen des oder der von der Verbindung der Formel (I) verschiedenen Riechstoffe mit einer Menge an Verbindung der Formel (I), die ausreicht, die natürliche Frische und/oder Ausstrahlung des oder der von der Verbindung der Formel (I) verschiedenen Riechstoffe zu verstärken und/oder fettige, technische und/oder metallische Noten zu maskieren oder zu vermindern.

Bevorzugt ist ein solches erfindungsgemäßes Verfahren, in dem der oder die von der Verbindung der Formel (I) verschiedene Riechstoffe ausgewählt sind aus den Bestandteilen (b) und/oder (c) einer erfindungsgemäßen Riechstoffmischung.

In solchen bevorzugten Verfahren ist vorzugsweise
- das Massenverhältnis der Gesamtmenge an Riechstoffen des Bestandteils (b) zur Verbindung der Formel (I) größer oder gleich 99 : 1 ist, bevorzugt größer oder gleich 99,9 : 0,1, besonders bevorzugt größer oder gleich 99,999 : 0,001 ist und/oder
- das Massenverhältnis der Gesamtmenge an Riechstoffen des Bestandteils (c) zur Verbindung der Formel (I) größer oder gleich 99 : 1 ist, bevorzugt größer oder gleich 99,9 : 0,1, besonders bevorzugt größer oder gleich 99,999 : 0,001 ist.

Die obigen Ausführungen zu bevorzugten erfindungsgemäßen Riechstoffmischungen gelten für die erfindungsgemäßen Verfahren entsprechend.

Die Erfindung betrifft auch ein parfümiertes Produkt enthaltend eine erfindungsgemäße Riechstoffmischung, vorzugsweise ein erfindungsgemäßes Parfümöl, wobei die Riechstoffmischung vorzugsweise in einer sensorisch wirksamen Menge in dem parfümierten Produkt enthalten ist.

"Sensorisch wirksame Menge" bedeutet im vorliegenden Zusammenhang, dass das erfindungsgemäße parfümierte Produkt im Betrieb bzw. bei Benutzung die sensorischen Eigenschaften der erfindungsgemäßen Riechstoffmischung erkennen lässt.

Bevorzugte erfindungsgemäße parfümierte Produkte sind ausgewählt aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Besonders bevorzugte erfindungsgemäße parfümierte Produkte sind ausgewählt aus folgender Liste:
- Eau de Parfums, Eau de Toilettes, Rasierwässer (After-shave), Eau de Colognes, Pre-shave-Produkte, Splash-Colognes;
- saure, alkalische und neutrale Reinigungsmittel, insbesondere im Haushaltsbereich, vorzugsweise Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäscheweichspüler, Oberflächendesinfektionsmittel, insbesondere für harte Oberflächen (hard surface cleaner);
- Körperpflegemittel, vorzugsweise feste und flüssige Seifen, Duschgele, Shampoos, Rasierseifen, Rasierschäume;
- kosmetische Emulsionen vom Öl-in-Wasser-, vom Wasser-in-Öl- und vom Wasser-in-Öl-in-Wasser-Typ, vorzugsweise Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und - lotionen, After-shave-Cremes und -lotionen, Hautbräunungscremes und -lotionen, Hautaufhellungscremes und -lotionen;
- Haarpflegeprodukte, vorzugsweise Haarsprays, Haargele, festigende Haarlotionen, Haarspülungen, permanente und semipermanente Haarfärbemittel, Haarwässer, Haarcremes und -lotionen;
- Deodorantien und Antiperspirantien, vorzugsweise Achselsprays, Roll-ons (vorzugsweise als alkoholische oder nicht-alkoholische Lösung, als Gel oder (Micro)Emulsion, Deosticks (Deo-stifte), Deocremes.

Besonders bevorzugte erfindungsgemäße parfümierte Produkte sind Wasch- und Reinigungsmittel, Hygiene- oder Pflegeprodukte, insbesondere im Bereich der Körper-und Haarpflege, der Kosmetik und des Haushalts.

Bevorzugte erfindungsgemäße parfümierte Produkte sind solche, bei denen der Anteil der erfindungsgemäßen Riechstoffmischung an dem parfümierten Produkt 0,01 bis 10 Gew.-%, bevorzugt 0,1 bis 5 Gew.-% und besonders bevorzugt 0,25 bis 3 Gew.-% beträgt, jeweils bezogen auf die Gesamtmasse des parfümierten Produktes. Dies gilt insbesondere für die vorstehend genannten bevorzugten Produkte.

Die Erfindung betrifft zudem auch ein Verfahren zur Herstellung eines parfümierten Produktes umfassend die Schritte:
i) Bereitstellen einer erfindungsgemäßen Riechstoffmischung oder Herstellen einer Riechstoffmischung nach einem erfindungsgemäßen Verfahren,
ii) Bereitstellen der weiteren Bestandteile des parfümierten Produktes und
iii) Inkontaktbringen der in Schritt ii) bereitgestellten weiteren Bestandteile des parfümierten Produkts mit einer sensorisch wirksamen Menge der in Schritt i) bereitgestellten Riechstoffmischung; wobei die Menge der Verbindung der Formel (I) ausreicht, um die natürliche Frische und/oder Ausstrahlung eines, mehrerer oder sämtlicher Riechstoffe der Bestandteile (b) und/oder (c) zu verstärken und/oder fettige, technische und/oder metallische Noten zu maskieren oder zu vermindern
oder
I) Bereitstellen der Bestandteile des parfümierten Produktes, die keine Bestandteile (a), (b) bzw. (c) einer erfindungsgemäßen Riechstoffmischung sind
II) Mischen der in Schritt I) bereitgestellten Bestandteile des parfümierten Produkts mit den Bestandteilen (b) und/oder (c) einer erfindungsgemäßen Riechstoffmischung, sodass eine Mischung resultiert, in welcher der oder die Bestandteile (b) und/oder (c) in einer sensorisch wirksamen Menge vorliegen,
III) Inkontaktbringen der in Schritt II) hergestellten Mischung mit einer Menge der Verbindung der Formel (I), wobei die Menge der Verbindung der Formel (I) ausreicht, um die natürliche Frische und/oder Ausstrahlung eines, mehrerer oder sämtlicher Riechstoffe der Bestandteile (b) und/oder (c) zu verstärken und/oder fettige, technische und/oder metallische Noten zu maskieren oder zu vermindern.

Schließlich betrifft die Erfindung auch die Verwendung der Verbindung der Formel (I) 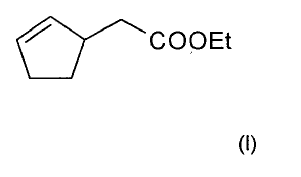 zum Verstärken der natürlichen Frische und/oder Ausstrahlung und/oder zum Maskieren oder Vermindern fettiger, technischer und/oder metallischer Noten eines oder mehrerer von der Verbindung der Formel (I) verschiedener Riechstoffe, insbesondere mit einer blumigen Geruchsnote, insbesondere Jasmin.

Bevorzugt ist eine erfindungsgemäße Verwendung der Verbindung der Formel (I), wobei der oder die von der Verbindung der Formel (I) verschiedenen Riechstoffe ausgewählt sind aus den Bestandteilen (b) und/oder (c) einer erfindungsgemäßen Riechstoffmischung.

Die nachfolgenden Beispiele erläutern die Erfindung; sofern nicht anders angegeben beziehen sich Anteile und Prozente auf das Gewicht.

### Verwendete Abkürzungen:

Dipropylenglycol (DPG), Diethylphthalat (DEP), Triethylcitrat (TEC), Isopropylmyristat (IPM); nat. = natürlich;
HEDIONE® HC/30 enthält mindestens 30 Gew.-% des cis-Isomers und knapp 70 Gew.-% des trans-Isomers von Methyldihydrojasmonat.

Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

### Beispiele:

### 1. Beispiel: Parfümöl P1

| Bevorzugte Anwendung: | **Allzweckreiniger 0.4%** |
|---|---|
| | |
| Aldehyde C 8 | 20,00 |
| Aldehyde C11 MOA | 10,00 |
| Limonenal | 2,00 |
| Mintonat | 120,00 |
| Dihydro Myrcenol | 50,00 |
| Lemon Oil | 10,00 |
| Litesea Cubeba Oil | 40,00 |
| Citronitrile | 10,00 |
| Eucalyptus Oil Citriodora | 50,00 |
| Citronella Oil | 40,00 |
| Orange Oil | 130,00 |
| Terpineol | 20,00 |
| Lavandin Oil | 50,00 |
| Rosemary Oil | 5,00 |
| Eucalyptus Oil | 5,00 |
| Aromabase Apple Green | 20,00 |
| Rosaphen® | 20,00 |
| Benzyl Acetate | 150,00 |
| Coumarin | 20,00 |
| Timberide | 2,00 |
| Dipropylene Glycol | 226,00 |
| | 1.000,00 |

Mit Zusatz von 0,5% einer 0,1%igen Lösung von Cyclopent-2-enyl-essigsäureethylester wird die Mischung runder, harmonischer, natürlicher, stärker und nicht mehr so technisch.

Mit Zusatz von 1% einer 0,1%igen Lösung von Cyclopent-2-enyl-essigsäureethylester wird die Mischung in der Top-Note stärker und ergibt eine zusätzliche mandarinen Note.

### 2. Beispiel: Parfümöl P2

| Bevorzugte | |
|---|---|
| Anwendung: | **Dusch-Gel 0.5%** |
| | |
| Limonenal 10% | 4,00 |
| Florazon | 1,00 |
| Leafovert® | 6,00 |
| Vertocitral | 14,00 |
| Phenylacetald. Dimethyl Acetal | 10,00 |
| Cyclogalbant® | 10,00 |
| Floropal | 7,00 |
| Mintonat | 50,00 |
| Lemongrass Oil Rect. | 8,00 |
| Orange Oil | 40,00 |
| Claritone® | 15,00 |
| Rosemary Oil | 5,00 |
| Artemisia Oil | 5,00 |
| Thyme Oil White | 1,00 |
| Pine Needle Oil | 4,00 |
| Hexyl Acetate | 20,00 |
| Ethyl Methyl Butyrate-2 | 2,00 |
| Allyl Cyclohexyl Propionate | 3,00 |
| Peach Total | 1,00 |
| Melon Concentrate | 12,00 |
| Lilax Soft | 100,00 |
| Linalool | 55,00 |
| Geranium RCO | 7,00 |
| Phenylethyl Alcohol | 60,00 |
| Geraniol Supra | 70,00 |
| Geranyl Acetate Pure | 20,00 |
| Benzyl Acetate | 24,00 |
| Hedione | 90,00 |
| Hexyl Cinnamic Aldehyde Alpha | 60,00 |
| Hexenyl Salicylate Cis-3 | 60,00 |
| Parmanyl® | 3,00 |
| Isoeugenol | 2,00 |
| Coumarin | 3,00 |
| Vetikolacetat® | 2,00 |
| Corps Racine 0.1 % | 7,00 |
| Evernyl 10% | 14,00 |
| Ambrettolide | 5,00 |
| Globanone® | 90,00 |
| Dipropylene Glycol | 110,00 |
| Triethyl Citrate | |
| | 1.000,00 |

Mit Zusatz von 0,5% einer 0,1%igen Lösung von Cyclopent-2-enyl-essigsäureethylester wird die Mischung runder, frischer.

Mit Zusatz von 1% einer 0,1%igen Lösung von Cyclopent-2-enyl-essigsäureethylester wirkt die Mischung mehr blumiger und stärker in Richtung weiße Blüte.

### 3. Beispiel: Parfümöl P3

| Bevorzugte Anwendung: | **Weichspüler 0.8%** |
|---|---|
| | |
| Aldehyde C11 Undecylenic 10% | 15,00 |
| Florazon | 10,00 |
| Mintonat | 10,00 |
| Dihydro Myrcenol | 40,00 |
| Mandaril 10% | 10,00 |
| Orange Oil | 70,00 |
| Nerolione 10% | 15,00 |
| Majantol® | 60,00 |
| Tetrahydro Linalool | 50,00 |
| Phenylethyl Alcohol | 70,00 |
| Citronellol 950 | 50,00 |
| Citronellyl Acetate Extra | 20,00 |
| Isodamascon® 10% | 10,00 |
| Benzyl Acetate | 30,00 |
| Hedione | 30,00 |
| Hexyl Cinnamic Aldehyde Alpha | 20,00 |
| Hexyl Salicylate | 100,00 |
| Eugenol | 10,00 |
| Heliotropin | 20,00 |
| Ethyl Vanillin 10% | 30,00 |
| Herbyl Propionate | 100,00 |
| Iso E Super | 100,00 |
| Patchouli Oil | 10,00 |
| Timberide | 5,00 |
| Corps Racine 0.1 % | 7,00 |
| Evernyl 10% | 5,00 |
| Galaxolide Type Base | 100,00 |
| Triethyl Citrate | 3,00 |
| | 1.000,00 |

Mit Zusatz von 0,5% einer 0,1%igen Lösung von Cyclopent-2-enyl-essigsäureethylester wird die Mischung runder, nicht mehr so technisch und mehr natürlicher.

Mit Zusatz von 1% einer 0,1%igen Lösung von Cyclopent-2-enyl-essigsäureethylester wirkt die Mischung mehr blumiger und stärker in Richtung weiße Blüte.

### 4. Beispiel: Parfümöl P4

| Bevorzugte Anwendung: | **Weichspüler 0.8%** |
|---|---|
| | |
| Aldehyde C12 | 5,00 |
| Alcohol C 6 | 10,00 |
| Dihydro Myrcenol | 30,00 |
| Claritone® | 10,00 |
| Petitgrain Oil Parag. | 5,00 |
| Methyl Naphtyl Ketone Beta Cryst | 10,00 |
| Lavandin Grosso | 20,00 |
| Rosemary Oil Tun. | 10,00 |
| Hexyl Acetate | 5,00 |
| Cyclamen Aldehyde | 10,00 |
| Mugetanol® | 40,00 |
| Majantol® | 30,00 |
| Tetrahydro Muguol | 20,00 |
| Base Muguet B | 20,00 |
| Tetrahydro Linalool | 40,00 |
| Dimethyl Benzyl Carbinyl Acetate | 30,00 |
| Terpineol Pure | 20,00 |
| Rose Oxide HC 10% | 20,00 |
| Phenyl Ethyl Alcohol | 60,00 |
| Base Rose Pamela-Y | 30,00 |
| Tetrahydrogeraniol | 10,00 |
| Isodamascon® 10% | 20,00 |
| Benzyl Acetone | 40,00 |
| Hexyl Salicylate | 140,00 |
| Parmanyl® 10% | 10,00 |
| Ionone Beta | 10,00 |
| Isoraldeine | 50,00 |
| Base Ylang B | 15,00 |
| Anisic Aldehyde Pure | 10,00 |
| Herbaflorat | 30,00 |
| Herbyl Proprionate | 60,00 |
| Iso E Super | 120,00 |
| Sandel 80 | 20,00 |
| Ysamber® K | 10,00 |
| Globanone® | 30,00 |
| Dipropylene glycol | |
| | 1000,00 |

Mit Zusatz von 0,5% einer 0,1%igen Lösung von Cyclopent-2-enyl-essigsäureethylester wird die Mischung runder, blumiger und geht in Richtung Lavendel.

Mit Zusatz von 1 % einer 0,1 %igen Lösung von Cyclopent-2-enyl-essigsäureethylester erhält die Mischung mehr Volumen und wirkt stärker in Richtung weiße Blüte.

### 5. Beispiel: Parfümöl P5

| Bevorzugte Anwendung:"**Body Lotion**" **(Körperlotion)** | **0.3%** |
|---|---|
| | |
| Aldehyd C14 SOG | 5,00 |
| Allylamylglycolat | 2,00 |
| Allylcyclohexylpropionat | 2,00 |
| Allylheptylat | 5,00 |
| Allylionone | 1,00 |
| Ambroxide 10%IPM | 4,00 |
| Benzyl acetat | 2,00 |
| Benzyl salicylat | 21,50 |
| Bourgeonal | 4,00 |
| Damascon alpha 10%DPG | 1,00 |
| Dihydromyrcenol | 20,00 |
| Dimethylbenzylcarbinylacetat | 10,00 |
| Farenal | 1,00 |
| Frambinon 10%DPG | 3,00 |
| Geranylacetat pur | 4,00 |
| Hedion | 200,00 |
| Indoflor krist 10%DPG | 5,50 |
| Ionone alpha | 10,00 |
| Iso e super | 40,00 |
| Jasmon cis | 1,00 |
| Leafovert | 2,00 |
| Macrolide supra | 30,00 |
| Majantol | 50,00 |
| Manzanate 10%IPM | 4,00 |
| Mayol | 55,00 |
| Methylphenylacetat 10%DPG | 2,00 |
| Mintonat | 5,00 |
| Nonadienal trans, cis-2,6 5%TEC 1 %DPG | 2,00 |
| Patchouli oel entf | 2,00 |
| Phenylethylalkohol | 15,00 |
| Phenylethyldimethylcarbinol | 7,00 |
| Rosaphen | 20,00 |
| Rosenoxid L 1%DPG | 5,00 |
| Sandranol | 5,00 |
| Tetrahydromuguol | 40,00 |
| Undecavertol | 2,00 |
| Veloutone 10%DPG | 3,00 |
| Vertocitral | 2,00 |
| Vertomugual | 1,00 |
| Dipropylene glycol | 406,00 |
| | |
| | 1.000,00 |

Mit Zusatz von 0,5% einer 0,1%igen Lösung von Cyclopent-2-enyl-essigsäureethylester wird die Mischung runder, frischer und mehr natürlicher.

Mit Zusatz von 1 % einer 0,1 %igen Lösung von Cyclopent-2-enyl-essigsäureethylester erhält die Mischung mehr Volumen und wirkt stärker in Richtung weiße Blüte (Jasmin).

### 6. Beispiel: Geruchsbeschreibung von bevorzugten Riechstoffen nach Zusatz von Cyclopent-2-enyl-essigsäureethylester

| **Riechstoffe** | **Typ** | **Massenverhältnis des Riechstoffes zur Verbindung der Formel (I) (0,1%ig in DPG)** | **Geruchsbeschreibung im Vergleich zum Geruch des reinen Riechstoffs** |
|---|---|---|---|
| Mugetanol | Alkohol | 100 : 0,5 | weicher, natürlicher, |
| (M=170) | | 100 : 0,1 | mehr blumiger, |
| Dihydromyrcenol (M=156) | Alkohol | 99:1,0 | weniger technisch, mehr natürlich-lavendelartig, mehr blumig |
| Linalool (M=154) | Alkohol | 99 : 1,0 | weniger technisch, frischer, natürlicher |
| Geraniol (M=154) | Alkohol | 99 : 1,0 | weniger fettig, weniger |
| | | | metallisch, runder, |
| | | | natürlicher, mehr Volumen |
| Citronellol | Alkohol | 100 : 0,5 | weniger fettig, mehr |
| (M=156) | | 100 : 1,0 | natürlich, mehr rosig |
| Phenoxanol | Alkohol | 100 : 1,0 | weniger technisch, |
| (M=178) | | 100 : 2,0 | mehr rosiger, natürlicher |
| Lilial® (M=204) | Aldehyd | 100 : 1,0 | weniger technisch, frischer, |
| | | | natürlicher, stärker nach |
| | | | Maiglöckchen |
| Aldehyd MNA | Aldehyd | 100 : 1,0 | weniger fettig, weniger |
| (M=184) | | | metallisch, runder |
| Melonal® (M=140) | Aldehyd | 100 : 1,0 | weniger technisch, weniger |
| | | | metallisch, runder, |
| | | | natürlicher |
| Hedion® (M=226) | Keton/Ester | 100 : 1,0 | weniger fettig, blumiger, runder, natürlicher, stärker nach Jasmin |
| para tert.-Butylcyclohexanon (M=154) | Keton | 100 : 1,0 | weniger technisch, runder, harmonischer |
| Oryclon® (M=198) | Ester | 100 : 1,0 | wenigertechnisch, runder, harmonischer, frischer |

### 7. Beispiel: Geruchsbeschreibung von bevorzugten Riechstoffmischungen nach Zusatz von Cyclopent-2-enyl-essigsäureethylester

| **Riechstoffe** | **Typ** | **Massenverhältnis von Alkohol zu Hedion** | **Geruchsbeschreibung in einer 0,1 %igen DPG-Lösung mit Zusatz von 1g Verbindung der Formel (I) pro 100 g des Alkohol/Hedion-Gemisches im Vergleich zum Geruch des reinen Riechstoffgemisches** |
|---|---|---|---|
| Mayol Hedion | Alkohol + Keton/Ester | 1:1 | weicher, natürlicher, mehr blumiger, jasminiger |
| Dihydromyrcenol Hedion | Alkohol + Keton/Ester | 3:1 | frischer, blumiger, lavendelartiger, natürlicher |

Die Parfumöle P1, P2, P3, P4 bzw. P5 aus den obigen Parfümöl-Beispielen 1 bis 5 wurden jeweils separat in die nachfolgenden Formulierungen eingearbeitet.

Die oben bei dem jeweiligen Parfümöl beschriebenen geruchlichen Effekte wurden jeweils auch in den nachfolgenden Formulierungen beobachtet.

### Formulierungsbeispiele

### Beispiel F1 - Waschpulver

| **Material** | **Chemischer Name** | **Funktion** | **Gew.-%** | **Gew.-%** |
|---|---|---|---|---|
| Natrium Metasilicat Pentahydrat | Sodium Metasilicate Pentahydrate | | Ad 100 | Ad 100 |
| Natriumhydrogen-carbonat | Sodium hydrogen carbonate | Alkali | 15,0 | 15,0 |
| Natrium-percarbonat | Sodium carbonate peroxyhydrate | Bleichmittel | 15,0 | 15,0 |
| Peractive AC Blue | TAED / Na-Carboxymethylcellulose | Aktivator | 5,00 | 5,00 |
| Genapol OA-080 | Oxoalkohol C14-15, 8EO | Nichtionisches Tensid | 3,00 | 3,00 |
| Texapon K12 Pulver | Sodium Lauryl Sulphate C12 | Anionisches Tensid | 7,00 | 7,00 |
| Tinopal CBS-X | | Aufheller | 0,50 | 0,50 |
| Savinase 6.0 T, Type W | Protease | Enzym | 0,40 | 0,40 |
| Termamyl 120 T | Alpha-Amylase | Enzym | 0,30 | 0,30 |
| Natriumsulfat | Sodium Sulphate | Füllstoff | 5,50 | 5,50 |
| Parfumöl P1, P2, P3, P4 bzw. P5 | | Parfum (Fragrance) | 0,30 | 0,50 |

### Beispiel F2 - Allzweckreiniger

| **Material** | **Chemischer Name** | **Funktion** | **Gew.-%** | **Gew.-%** |
|---|---|---|---|---|
| Entionisiertes Wasser | Water | Lösungsmittel | Ad 100 | Ad 100 |
| Mergal K9N | 5-Chloro-2-methyl-3-(2H)-isothiazolone und 2-methyl-3-(2H)-isothiazolone | Konservierungsmittel | 0,1 | 0,1 |
| Trinatriumcitrat Dihydrat | Tri Sodium Citrate Dihydrate | Komplexbildner | 3,0 | 3,0 |
| Zetesol NL-2 | Fatty alcohol C12-14-sulfate, Sodium | Anionisches Tensid | 30,0 | 30,0 |
| Imbentin C/125/055 | Fatty alcohol C12-C15, 8EO | Nichtionisches Tensid | 5,0 | 5,0 |
| Ethanol | Ethanol | Lösungsmittel | 2,0 | 2,0 |
| Parfumöl P1, P2, P3, P4, bzw. P5 | | Parfum (Fragrance) | 0,3 | 0,5 |

### Beispiel F3 - Shampoo

| **Material** | **INCI-Name** | **Gew.-%** | **Gew.-%** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butylparaben, Propylparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumöl P1, P2, P3, P4, bzw. P5 | Parfum (Fragrance) | 0,5 | 0,8 |

### Beispiel F4 - Duschgel

| **Material** | **INCI-Name** | **Gew.-%** | **Gew.-%** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butylparaben, Propylparaben, Isobutylparaben | 0,5 | 0,5 |
| Natriumchlorid | Sodium Chloride | 1,4 | 1,4 |
| Citronensäure Monohydrat kristallin | Citric Acid | 1,3 | 1,3 |
| Parfumöl P1, P2, P3, P4, bzw. P5 | Parfum (Fragrance) | 0,5 | 0,7 |

### Beispiel F5 - Weichspüler

| **Material** | **Chemischer Name** | **Funktion** | **Gew.-%** | **Gew.-%** |
|---|---|---|---|---|
| Entionisiertes Wasser | Wasser | Lösungsmittel | Ad 100 | Ad 100 |
| Rewoquat WE 18 | Dialkylesterammoniumethosulfate | Kationisches Tensid | 16,6 | 16,6 |
| Mergal K9N | 5-Ch loro-2-methyl-3-(2H)-isothiazolone und 2-methyl-3-(2H)-isothiazolone | Konservierungs-mittel | 0,10 | 0,10 |
| Dow Corning 1520 Antifoam | Polydimethyl-siloxane | Entschäumer | 0,30 | 0,30 |
| Magnesium Chlorid 1%ige Lösung | Magnesium Chlorid Lösung | Konsistenzgeber | 10,00 | 10,00 |
| Parfumöl P1, P2, P3, P4, bzw. P5 | | Fragrance | 0,55 | 0,75 |

### Beispiel F6 - Eau de Cologne / Eau de Toilette

| **Inhaltsstoffe** | **Gew.-%** | **Gew.-%** |
|---|---|---|
| Parfumöl P1, P2, P3, P4, bzw. P5 | 5 | 10 |
| Ethanol | Ad 100 | Ad 100 |
| Wasser | 18 | 10 |

### Beispiel F7 - Aerosol-Pumpspray

| **Inhaltsstoffe** | **Gew.-%** | **Gew.-%** |
|---|---|---|
| Parfumöl P1, P2, P3, P4, bzw. P5 | 1,0 | 1,5 |
| Ethanol | Ad 100 | Ad 100 |
| Wasser | 5,0 | 8,0 |
| Alpha-Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel F8 - Shampoo

| **Inhaltsstoffe** | **Gew.-%** | **Gew.-%** | **Gew.-%** |
|---|---|---|---|
| Natriumlaurylethersulfat (z.B. Texapon NSO, Fa. Cognis Deutschland GmbH) | 12 | 12 | 12 |
| Cocamidopropylbetain (z.B. Dehyton K, Fa. Cognis Deutschland GmbH) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl-und Propylparaben | 0,5 | 0,5 | 0,5 |
| Parfumöl P1, P2, P3, P4, bzw. P5 | 0,3 | 0,5 | 0,7 |
| Wasser | Ad 100 | Ad 100 | Ad 100 |

### Beispiel F9 - Waschpulver

| **Inhaltsstoffe** | **Gew.-%** | **Gew.-%** | **Gew.-%** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellulose | 1,2 | 1,2 | 1,2 |
| Dequest 2066([[(Phosphonomethyl)imino]bis[(ethylennitrilo)bis (methylen)]]tetrakis-phosphonsäure, Natriumsalz) | 2,8 | 2,8 | 2,8 |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfumöl P1, P2, P3, P4, bzw. P5 | 0,25 | 0,35 | 0,5 |
| EDTA | 1,0 | 1,0 | 1,0 |

### Beispiel F10 - Flüssigwaschmittel

| **Inhaltsstoffe** | **Gew.-%** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfumöl P1, P2, P3, P4, bzw. P5 | 0,5 |

### Beispiel F11 - Flüssigwaschmittel Konzentrat

| **Inhaltsstoffe** | **Gew.-%** |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfumöl P1, P2, P3, P4, bzw. P5 | 0,7 |

## Patentansprüche

1. Riechstoffmischung, vorzugsweise Parfümöl, umfassend die Bestandteile
(a) Verbindung der Formel (I) 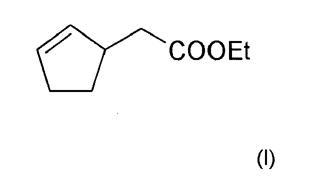 sowie zusätzlich:
(b) einen oder mehrere Riechstoffe, vorzugsweise mit einer blumigen Geruchsnote, aus der Gruppe bestehend aus Alkoholen und Aldehyden mit einer Molmasse von 210 g/mol oder weniger
und/oder
(c) einen oder mehrere Riechstoffe aus der Gruppe bestehend aus Ketonen, Ethern und Estern mit einer Molmasse im Bereich von 190 g/mol bis 250 g/mol.

2. Riechstoffmischung, vorzugsweise Parfümöl, nach Anspruch 1, **dadurch gekennzeichnet, dass** der Bestandteil (b) zwei, drei, vier, fünf oder mehr verschiedene Riechstoffe enthält
und/oder
dass der Bestandteil (c) zwei, drei, vier, fünf oder mehr verschiedene Riechstoffe enthält.

3. Riechstoffmischung, vorzugsweise Parfümöl, nach Anspruch 1 oder 2, **dadurch gekennzeichnet, dass** das Massenverhältnis der Gesamtmenge an Riechstoffen des Bestandteils (b) zur Verbindung der Formel (I) größer oder gleich 99 : 1 ist, bevorzugt größer oder gleich 99,9 : 0,1, besonders bevorzugt größer oder gleich 99,999 : 0,001 ist
und/oder
dass das Massenverhältnis der Gesamtmenge an Riechstoffen des Bestandteils (c) zur Verbindung der Formel (I) größer oder gleich 99 : 1 ist, bevorzugt größer oder gleich 99,9 : 0,1, besonders bevorzugt größer oder gleich 99,999 : 0,001 ist.

4. Riechstoffmischung, vorzugsweise Parfümöl, nach einem der vorherigen Ansprüche, wobei der, mehrere oder sämtliche Riechstoffe des Bestandteils (b) jeweils eine Molmasse im Bereich von 140 bis 170 g/mol aufweisen.

5. Riechstoffmischung, vorzugsweise Parfümöl, nach einem der vorhergehenden Ansprüche, wobei der oder die Riechstoffe des Bestandteils (c) ausgewählt sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, Oxacyclohexa-decan-2-on, 15-Hydroxy-Pentadecanonsäurelacton, 5-Cyclohexadecen-1-on, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 8-Cyclohexadecen-1-on, 3a,6,6,9a-Tetramethyl dodecahydronaphtho[2,1-b]furan, alpha-Iron, beta-Iron, alpha-n-Methylionon, beta-n-Methylionon, alpha-Isomethylionon, beta-Isomethylionon und Allylionon
und/oder
der oder die Riechstoffe des Bestandteils (b) ausgewählt sind aus der Gruppe bestehend aus 2-Methyl-3-(4-tert-butylphenyl)propanal, 4-(4-Hydroxy-4-methylpentyl)-3-cyclohexencarboxaldehyd, 2-Methyl-4-(2,2,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

6. Riechstoffmischung, vorzugsweise Parfümöl, nach einem der vorhergehenden Ansprüche, **dadurch gekennzeichnet, dass** Bestandteil (c) Methyldihydrojasmonat enthält oder aus Methyldihydrojasmonat besteht
und vorzugsweise der Gehalt an cis-Methyldihydrojasmonat mehr als 30 Gew.-%, weiter bevorzugt mehr als 60 Gew.-%, besonders bevorzugt mehr als 75 Gew.-%, ganz besonders bevorzugt mehr als 90 Gew.-% beträgt, jeweils bezogen auf die Gesamtmenge an cis- und trans-Methyldihydrojasmonat.

7. Verfahren zur Herstellung einer Riechstoffmischung, vorzugsweise eines Parfümöls, nach einem der vorhergehenden Ansprüche, **gekennzeichnet durch** folgenden Schritt:
- Mischen des Bestandteils (a) mit Bestandteil (b) und/oder (c).

8. Verfahren nach Anspruch 7, wobei eine Riechstoffmischung, vorzugsweise ein Parfümöl, resultiert, in der
- das Massenverhältnis der Gesamtmenge an Riechstoffen des Bestandteils (b) zur Verbindung der Formel (I) größer oder gleich 99 : 1 ist, bevorzugt größer oder gleich 99,9 : 0,1, besonders bevorzugt größer oder gleich 99,999 : 0,001 ist,
und/oder
- das Massenverhältnis der Gesamtmenge an Riechstoffen des Bestandteils (c) zur Verbindung der Formel (I) größer oder gleich 99 : 1 ist, bevorzugt größer oder gleich 99,9 : 0,1, besonders bevorzugt größer oder gleich 99,999 : 0,001 ist.

9. Verfahren zum Verstärken der natürlichen Frische und/oder Ausstrahlung und/oder zum Maskieren oder Vermindern fettiger, technischer und/oder metallischer Noten eines oder mehrerer von der Verbindung der Formel (I) verschiedener Riechstoffe, insbesondere eines oder mehrerer von der Verbindung der Formel (I) verschiedener Riechstoffe mit einer blumigen Geruchsnote, insbesondere Jasmin, umfassend den folgenden Schritt:
- Vermischen des oder der von der Verbindung der Formel (I) verschiedenen Riechstoffe mit einer Menge an Verbindung der Formel (I), die ausreicht, die natürliche Frische und/oder Ausstrahlung des oder der von der Verbindung der Formel (I) verschiedenen Riechstoffe zu verstärken und/oder fettige, technische und/oder metallische Noten zu maskieren oder zu vermindern.

10. Verfahren nach Anspruch 9, **dadurch gekennzeichnet, dass** der oder die von der Verbindung der Formel (I) verschiedenen Riechstoffe ausgewählt sind aus den Bestandteilen (b) und/oder (c) einer Riechstoffmischung nach einem der Ansprüche 1 bis 6.

11. Verfahren nach Anspruch 10, wobei
- das Massenverhältnis der Gesamtmenge an Riechstoffen des Bestandteils (b) zur Verbindung der Formel (I) größer oder gleich 99 : 1 ist, bevorzugt größer oder gleich 99,9 : 0,1, besonders bevorzugt größer oder gleich 99, 999 : 0,001 ist
und/oder
- das Massenverhältnis der Gesamtmenge an Riechstoffen des Bestandteils (c) zur Verbindung der Formel (I) größer oder gleich 99 : 1 ist, bevorzugt größer oder gleich 99,9 : 0,1, besonders bevorzugt größer oder gleich 99,999 : 0,001 ist.

12. Parfümiertes Produkt enthaltend eine Riechstoffmischung, vorzugsweise ein Parfümöl, nach einem der Ansprüche 1 bis 6, vorzugsweise in einer sensorisch wirksamen Menge.

13. Parfümiertes Produkt nach Anspruch 12, wobei das Produkt ausgewählt ist aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen,
flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und - lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe
und/oder
der Anteil der Riechstoffmischung nach einem der Ansprüche 1 bis 6 an dem parfümierten Produkt 0,01 bis 10 Gew.-%, bevorzugt 0,1 bis 5 Gew.-% und besonders bevorzugt 0,25 bis 3 Gew.-% beträgt, jeweils bezogen auf die Gesamtmasse des parfümierten Produktes.

14. Verfahren zum Herstellen eines parfümierten Produktes umfassend die Schritte:
i) Bereitstellen einer Riechstoffmischung nach einem der Ansprüche 1 bis 6 oder Herstellen einer Riechstoffmischung nach einem Verfahren nach einem der der Ansprüche 7 bis 8,
ii) Bereitstellen der weiteren Bestandteile des parfümierten Produktes und
iii) Inkontaktbringen der in Schritt ii) bereitgestellten weiteren Bestandteile des parfümierten Produkts mit einer sensorisch wirksamen Menge der in Schritt i) bereitgestellten Riechstoffmischung; wobei die Menge der Verbindung der Formel (I) ausreicht, um die natürliche Frische und/oder Ausstrahlung eines, mehrerer oder sämtlicher Riechstoffe der Bestandteile (b) und/oder (c) zu verstärken und/oder fettige, technische und/oder metallische Noten zu maskieren oder zu vermindern
oder
I) Bereitstellen der Bestandteile des parfümierten Produktes, die keine Bestandteile (a), (b) bzw. (c) einer Riechstoffmischung sind wie in einem der Ansprüche 1 bis 6 definiert
II) Mischen der in Schritt I) bereitgestellten Bestandteile des parfümierten Produkts mit den Bestandteilen (b) und/oder (c) einer Riechstoffmischung wie in einem der Ansprüche 1 bis 6 definiert, sodass eine Mischung resultiert, in welcher der oder die Bestandteile (b) und/oder (c) in einer sensorisch wirksamen Menge vorliegen,
III) Inkontaktbringen der in Schritt II) hergestellten Mischung mit einer Menge der Verbindung der Formel (I), wobei die Menge der Verbindung der Formel (I) ausreicht, um die natürliche Frische und/oder Ausstrahlung eines, mehrerer oder sämtlicher Riechstoffe der Bestandteile (b) und/oder (c) zu verstärken und/oder fettige, technische und/oder metallische Noten zu maskieren oder zu vermindern.

15. Verwendung der Verbindung der Formel (I) 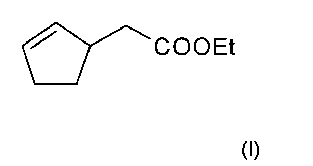 zum Verstärken der natürlichen Frische und/oder Ausstrahlung und/oder zum Maskieren oder Vermindern fettiger, technischer und/oder metallischer Noten eines oder mehrerer von der Verbindung der Formel (I) verschiedener Riechstoffe, insbesondere mit einer blumigen Geruchsnote, insbesondere Jasmin,
wobei vorzugsweise
der oder die von der Verbindung der Formel (I) verschiedenen Riechstoffe ausgewählt sind aus den Bestandteilen (b) und/oder (c) einer Riechstoffmischung nach einem der Ansprüche 1 bis 6.

## Claims

1. Fragrance mixture, preferably perfume oil, comprising the components
(a) compound of formula (I) 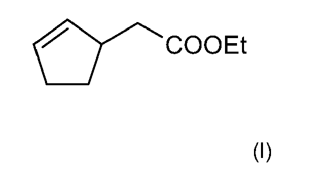 and additionally:
(b) one or more fragrances, preferably with a flowery scent, from the group consisting of alcohols and aldehydes with a molar mass of 210 g/mol or less
and/or
(c) one or more fragrances from the group consisting of ketones, ethers and esters with a molar mass in the range from 190 g/mol to 250 g/mol.

2. Fragrance mixture, preferably perfume oil, according to claim 1, **characterized in that** component (b) comprises two, three, four, fife or more different fragrances and/or
that component (c) comprises two, three, four, fife or more different fragrances.

3. Fragrance mixture, preferably perfume oil, according to claim 1 or 2, **characterized in that** the mass ratio of the total amount of fragrances of component (b) to compound of formula (I) is greater or equal 99 : 1, preferably greater or equal 99,9 : 0,1, particularly preferably greater or equal 99,999 : 0,001
and/or
that the mass ratio of the total amount of fragrances of component (c) to compound of formula (I) is greater or equal 99 : 1, preferably greater or equal 99,9 : 0,1, particularly preferably greater or equal 99,999 : 0,001.

4. Fragrance mixture, preferably perfume oil, according to one of the preceding claims, wherein the, several or all fragrance(s) of component (b) each have a molar mass in the range from 140 to 170 g/mol.

5. Fragrance mixture, preferably perfume oil, according to one of the preceding claims, wherein the fragrance(s) of component (c) is/are selected from the group consisting of methyl dihydrojasmonate, benzyl salicylate, cis-3-hexenyl salicylate, isoamyl salicylate, hexyl salicylate, 2,3,8,8-tetramethyl-1,2,3,4,5,6,7,8-octahydro-2-naphthalenyl methyl ketone, linalyl acetate, ethyl linalyl acetate, cedryl methyl ether, cedryl methyl ketone, cedryl acetate, (4aR,5R,7aS,9R)-octahydro-2,2,5,8,8,9a-hexamethyl-4H-4a,9-methano azuleno(5,6-d)-1,3-dioxol), 1',1',5',5'-tetra methyl hexahydro spiro[1.3-dioxolan-2.8'(5'H)-2H-2.4a] methanonaphthaline, cyclo dodecyl methylether, (ethoxymethoxy) cyclododecane, decahydro-beta-naphthyl acetate, 4,7-methano-3a,4,5,6,7,7a-hexahydro-5(-6)-indenyl acetate, allyl-3-cyclohexyl propionate, allyl cyclohexyl oxyacetate, benzyl benzoate, benzyl cinnamate, oxacyclohexadecane-2-one, 15-hydroxy pentadecanonic acid lactone, 5-cyclohexadecen-1-one, 3-methyl cyclopentadecenone, 1,3,4,6,7,8-hexahydro-4,6,6,7,8,8-hexamethyl cyclopenta[g]-2-benzopyran, 2-[1-(3,3-dimethyl cyclohexyl) ethoxy]-2-methyl-,1-propanoate, 1,4-dioxacycloheptadecane-5,17-dione, 3-methy cyclopentadecanone, 8-cyclohexadecene-1-one, 3a,6,6,9a-tetramethyl dodecahydronaphtho[2,1-b]furane, alpha-iron, beta-iron, alpha-n-methyl ionone, beta-n-methyl ionone, alpha-isomethyl ionone, beta-Isomethyl ionone and allyl ionone
and/or
the fragrance(s) of component (b) is/are selected from the group consisting of 2-methyl-3-(4-tert-butylphenyl) propanal, 4-(4-hydroxy-4-methylpentyl)-3-cyclohexene carboxaldehyde, 2-methyl-4-(2,2,6-trimethyl-1-cyclohexene-1-yl)-2-butenal, 1-methyl-4-(4-methyl-3-pentene-1-yl)-3-cyclohexene carboxaldehyde, 3-(3-isopropyl-phenyl)-butyraldehyde, (E)-2,6,10-trimethyl undeca-5,9-dienal, Benzo[1,3]dioxole-5-carbaldehyde, 2,2-dimethyl-3-phenyl propane-1-ol, 2,2-dimethyl-3-m-tolyl propane-1-ol, 1-(4-isopropyl-cyclohexyl) ethanol, (4-isopropyl cyclohexyl) methanol, 2-phenyl ethanol, 2-isobutyl-4-methyl tetrahydropyrane-4-ol, 3,7-dimethyl octa-1,6-diene-3-ol, (Z)-3,7-dimethyl octa-2,6-diene-1-ol, (E)-3,7-dimethyl octa-2,6-diene-1-ol, 3,7-dimethyl oct-6-ene-1-ol, 2,6-dimethyl oct-7-ene-2-ol, 3,7-dimethyl octane-1-ol, 2-methyl-6-methylen oct-7-ene-2-ol and (E/Z)-3,7-dimethyl nona-1,6-diene-3-ol.

6. Fragrance mixture, preferably perfume oil, according to one of the preceding claims, **characterized in that** component (c) comprises methyl dihydrojasmonate or consists of methyl dihydrojasmonate
and preferably the content of cis-methyl dihydrojasmonate is more that 30 wt.-%, further preferably more than 60 wt.-%, particularly preferably more than 75 wt.-%, especially preferably more than 90 wt.-%, in each case with respect to the total amount of cis- and trans-methyl dihydrojasmonate.

7. Method for producing a fragrance mixture, preferably a perfume oil, according to one of the preceding claims, **characterized by** the following step:
- mixing of component (a) with component (b) and/or (c).

8. Method according to claim 7, wherein a fragrance mixture, preferably a perfume oil is obtained, in which
- the mass ratio of the total amount of fragrances of component (b) to compound of formula (I) is greater or equal 99 : 1, preferably greater or equal 99,9 : 0,1, particularly preferably greater or equal 99,999 : 0,001,
and/or
- the mass ratio of the total amount of fragrances of component (c) to compound of formula (I) is greater or equal 99 : 1, preferably greater or equal 99,9 : 0,1, particularly preferably greater or equal 99,999 : 0,001.

9. Method for enhancing the natural freshness and/or effectiveness and/or for masking or decreasing fatty, technical and/or metallic notes of one or more fragrances other than the compound of formula (I), in particular of one or more fragrance(s) other than the compound of formula (I) with a flowery scent, in particular jasmine, comprising the following step:
- mixing the fragrance(s) other than the compound of formula (I) with an amount of compound of formula (I) sufficient to enhance the natural freshness and/or effectiveness of the fragrance(s) other than the compound of formula (I) and/or to mask or decrease fatty, technical and/or metallic notes.

10. Method according to claim 9, **characterized in that** the fragrance(s) other than the compound of formula (I) are selected from the components (b) and/or (c) of a fragrance mixture according to one of claims 1 to 6.

11. Method according to claim 10, wherein
- the mass ratio of the total amount of fragrances of component (b) to compound of formula (I) is greater or equal 99 : 1, preferably greater or equal 99,9 : 0,1, particularly preferably greater or equal 99,999 : 0,001,
and/or
- the mass ratio of the total amount of fragrances of component (c) to compound of formula (I) is greater or equal 99 : 1, preferably greater or equal 99,9 : 0,1, particularly preferably greater or equal 99,999 : 0,001.

12. Perfumed product comprising a fragrance mixture, preferably a perfume oil, according to one of claims 1 to 6, preferably in a sensorially effective amount.

13. Perfumed product according to claim 12, wherein the product is selected from the group consisting of:
perfume extracts, eau de parfums, eau de toilettes, after-shave, eau de colognes, pre-shave-products, splash colognes, perfumed wet wipes, acidic, alkalic and neutral cleaning agents, textile refreshener, iron aids, liqid laundry soaps, laundry soap powders, laundry pretreatment agents, fabric softerners, cleaning soaps, cleaning tabs, disinfectants, surface disinfectants, air improver, aerosol sprays, waxes and polishes, body care products, hand crèmes and lotions, foot crèmes and lotions, hair removal crèmes and lotions, aftershave crèmes and lotions, tanning crèmes and lotions, hair care products, deodorants and antiperspirants, products of decorative cosmetics, candles, lamp oils, incense sticks, insecticides, repellents and blowing agents
and/or
the portion of the fragrance mixture according to one of claims 1 to 6 in the perfumed product is 0,01 to 10 wt.-%, preferably 0,1 to 5 wt.-% and particularly preferably 0,25 to 3 wt.-%, in each case with respect of the total mass of the perfumed product.

14. Method for producing a perfumed product comprising the steps:
i) providing a fragrance mixture according to one of claims 1 to 6 or producing a fragrance mixture by a method according to one of claims 7 to 8,
ii) providing the further components of the perfumed product and
iii) contacting the further components of the perfumed product provided in step ii) with a sensorially effective amount of the fragrance mixture provided in step i); wherein the amount of the compound of formula (I) is sufficient to enhance the natural freshness and/or effectiveness of one, several or all of the fragrances of component (b) and/or (c) and/or to mask or decrease fatty, technical and/or metallic notes
or
I) providing the components of the perfumed product, which are not (a), (b) or (c), respectively, of a fragrance mixture as defined in one of claims 1 to 6
II) mixing of the components of the perfumed product provided in step I) with the components (b) and/or (c) of a fragrance mixture as defined in one of claims 1 to 6, so that a mixture results, in which the components (b) and/or (c) are present in a sensorially effective amount,
III) contacting the mixture produced in step II) with an amount of the compound of formula (I) wherein the amount of the compound of formula (I) is sufficient to enhance the natural freshness and/or effectiveness of one, several or all of the fragrance(s) of component (b) and/or (c) and/or to mask or decrease fatty, technical and/or metallic notes.

15. Use of the compound of formula (I) 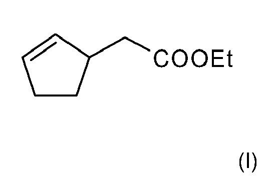 for enhancing the natural freshness and/or effectiveness and/or for masking or decreasing fatty, technical and/or metallic notes of one or more fragrances other than the compound of formula (I), in particular with a flowery scent, in particular jasmine,
wherein preferably
the fragrance(s) other than the compound of formula (I) are selected from the components (b) and/or (c) of a fragrance mixture according to one of claims 1 to 6.

## Revendications

1. Mélange de substances odoriférantes, de préférence huile de parfum, comprenant les composants
(a) composé de la formule (I) 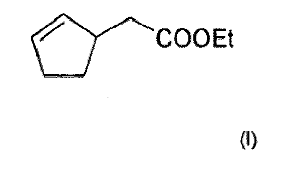 ainsi que, en sus :
(b) une ou plusieurs substance(s) odoriférante(s), de préférence ayant une note olfactive fleurie, provenant du groupe constitué par les alcools et les aldéhydes ayant une masse molaire de 210 g/mol ou moins et/ou
(c) une ou plusieurs substance(s) odoriférante(s) provenant du groupe constitué par les cétones, les éthers et les esters ayant une masse molaire comprise entre 190 g/mol et 250 g/mol.

2. Mélange de substances odoriférantes, de préférence huile de parfum, selon la revendication 1, **caractérisé par le fait que** ledit composant (b) contient deux, trois, quatre, cinq substances odoriférantes différentes ou plus
et/ou
que ledit composant (c) contient deux, trois, quatre, cinq substances odoriférantes différentes ou plus.

3. Mélange de substances odoriférantes, de préférence huile de parfum, selon la revendication 1 ou 2, **caractérisé par le fait que** le rapport de masse de la quantité totale de substances odoriférantes du composant (b) au composé de la formule (I) est supérieur ou égal à 99 : 1, de préférence supérieur ou égal à 99,9:0,1, de manière particulièrement préférée supérieur ou égal à 99,999 : 0,001,
et/ou
que le rapport de masse de la quantité totale de substances odoriférantes du composant (c) au composé de la formule (I) est supérieur ou égal à 99 : 1, de préférence supérieur ou égal à 99,9 : 0,1, de manière particulièrement préférée supérieur ou égal à 99,999 : 0,001.

4. Mélange de substances odoriférantes, de préférence huile de parfum, selon l'une quelconque des revendications précédentes, dans lequel ladite, plusieurs ou toutes les substances odoriférantes du composant (b) présentent chacune une masse molaire comprise entre 140 et 170 g/mol.

5. Mélange de substances odoriférantes, de préférence huile de parfum, selon l'une quelconque des revendications précédentes, dans lequel ladite ou les substance(s) odoriférante(s) du composant (c) sont choisies dans le groupe constitué par le dihydrojasmonate de méthyle, le salicylate de benzyle, le salicylate de cis-3-hexényle, le salicylate d'isoamyle, le salicylate d'hexyle, la 2,3,8,8-tétraméthyl-1,2,3,4,5,6,7,8-octahydro-2-naphtalénylméthylcétone, l'acétate de linalyle, l'acétate d'éthyl linalyle, l'éther méthylique de cédryle, la cédryl méthyl cétone, l'acétate de cédryle, le (4aR,5R,7aS,9R)-octahydro-2,2,5,8,8,9a-hexaméthyl-4H-4a,9-méthano azuléno(5,6-d)-1,3-dioxol), le 1',1',5',5'-tétra méthyl-hexahydrospiro[1.3-dioxolan-2.8'(5'H)-2H-2.4a]methanonaphtalène, le cyclododécyl méthyl éther, l'(éthoxyméthoxy)cyclododécane, le décahydro-bêta-naphthyl acétate, le 4,7-méthano-3a,4,5,6,7,7a-hexahydro-5(-6)-indénylacétate, l'allyl-3-cyclohexyl propionate, l'allyl cyclohexyl oxyacétate, le benzoate de benzyle, le cinnamate de benzyle, l'oxacyclohexa-décane-2-one, la lactone d'acide 15-hydroxy-pentadécanoïque, le 5-cyclohexadécène-1-one, le 3-méthylcyclopentadécenone, le 1,3,4,6,7,8-hexahydro-4,6,6,7,8,8-hexaméthyl cyclopenta[g]-2-benzopyrane, 2-[1-(3,3-diméthylcyclohexyl)éthoxy]-2-méthyl-,1-propanoate, le 1,4-dioxacycloheptadécan-5,17-dione, le 3-méthycyclopentadécanone, le 8-cyclohexadécène-1-one, le 3a,6,6,9a-tétraméthyl dodécahydronaphto[2,1-b]furane, l'alpha-irone, la bêta-irone, l'alpha-n-méthylionone, la bêta-n-méthylionone, l'alpha-isométhylionone, la bêtaisométhylionone et l'allylionone
et/ou
la ou les substance(s) odoriférante(s) du composant (b) sont choisies dans le groupe constitué par le 2-méthyl-3-(4-tert-butylphényl)propanal, le 4-(4-hydroxy4-méthylpentyl)-3-cyclohexène carboxaldehyde, le 2-méthyl-4-(2,2,6-triméthyl-1-cyclohexène-1-yl)-2-buténal, le 1-méthyl-4-(4-méthyl-3-pentène-1-yl)-3-cyclohexène carboxaldehyde, le 3-(3-isopropyl-phényl) butyraldehyde, le (E)-2,6,10-triméthyl-undéca-5,9-diénal, le benzo[1,3]dioxole-5-carbaldéhyde, le 2,2-diméthyl-3-phényl-propane-1-ol, le 2,2-diméthyl-3-m-tolyl-propane-1-ol, le 1-(4-isopropyl-cyclohexyl)-éthanol, le (4-isopropyl-cyclohexyl)-méthanol, le 2-phényléthanol, le 2-isobutyl-4-méthyl-tétrahydropyrane-4-ol, le 3,7-diméthyl-octa-1,6-diène-3-ol, le (Z)-3,7-diméthyl-octa-2,6-diène-1-ol, le (E)-3,7-diméthyl-octa-2,6-diène-1-ol, le 3,7-diméthyl-oct-6-ène-1-ol, le 2,6-diméthyl-oct-7-ène-2-ol, le 3,7-diméthyl-octane-1-ol, le 2-méthyl-6-méthylène-oct-7-ène-2-ol et le (E/Z)-3,7-diméthyl-nona-1,6-diène-3-ol.

6. Mélange de substances odoriférantes, de préférence huile de parfum, selon l'une quelconque des revendications précédentes, **caractérisé par le fait que** ledit composant (c) contient du dihydrojasmonate de méthyle ou se compose de dihydrojasmonate de méthyle
et que, de préférence, la teneur en cis-dihydrojasmonate de méthyle est supérieure à 30 % en poids, encore de préférence supérieure à 60 % en poids, de manière particulièrement préférée supérieure à 75 % en poids, de manière tout à fait préférée supérieure à 90 % en poids, respectivement par rapport à la quantité totale de cis- et trans- dihydrojasmonate de méthyle.

7. Procédé de préparation d'un mélange de substances odoriférantes, de préférence d'une huile de parfum, selon l'une quelconque des revendications précédentes, **caractérisé par** l'étape suivante :
- mélanger ledit composant (a) audit composant (b) et/ou (c).

8. Procédé selon la revendication 7, d'où résulte un mélange de substances odoriférantes, de préférence une huile de parfum, dans lequel
- le rapport de masse de la quantité totale de substances odoriférantes du composant (b) au composé de la formule (I) est supérieur ou égal à 99 : 1, de préférence supérieur ou égal à 99,9 : 0,1, de manière particulièrement préférée supérieur ou égal à 99,999 : 0,001,
et/ou
- le rapport de masse de la quantité totale de substances odoriférantes du composant (c) au composé de la formule (I) est supérieur ou égal à 99 : 1, de préférence supérieur ou égal à 99,9 : 0,1, de manière particulièrement préférée supérieur ou égal à 99,999 : 0,001.

9. Procédé destiné à intensifier la fraîcheur naturelle et/ou le rayonnement et/ou à masquer ou à réduire des notes graisseuses, techniques et/ou métalliques d'une ou de plusieurs substance(s) odoriférante(s) différente(s) du composé de la formule (I), en particulier d'une ou de plusieurs substance(s) odoriférante(s) différente(s) du composé de la formule (I) et ayant une note olfactive fleurie, en particulier le jasmin, comprenant l'étape suivante :
- mélanger ladite ou lesdites substance(s) odoriférante(s) différente(s) du composé de la formule (I), à une quantité de composé de la formule (I) qui est suffisante pour intensifier la fraîcheur naturelle et/ou le rayonnement de la ou des substance(s) odoriférante(s) différente(s) du composé de la formule (I) et/ou pour masquer ou pour réduire des notes graisseuses, techniques et/ou métalliques.

10. Procédé selon la revendication 9, **caractérisé par le fait que** ladite ou lesdites substance(s) odoriférante(s) différente(s) du composé de la formule (I) sont choisies dans les composants (b) et/ou (c) d'un mélange de substances odoriférantes selon l'une quelconque des revendications 1 à 6.

11. Procédé selon la revendication 10, dans lequel
- le rapport de masse de la quantité totale de substances odoriférantes du composant (b) au composé de la formule (I) est supérieur ou égal à 99 : 1, de préférence supérieur ou égal à 99,9 : 0,1, de manière particulièrement préférée supérieur ou égal à 99,999 : 0,001,
et/ou
- le rapport de masse de la quantité totale de substances odoriférantes du composant (c) au composé de la formule (I) est supérieur ou égal à 99 : 1, de préférence supérieur ou égal à 99,9 : 0,1, de manière particulièrement préférée supérieur ou égal à 99,999 : 0,001.

12. Produit parfumé contenant un mélange de substances odoriférantes, de préférence une huile de parfum, selon l'une quelconque des revendications 1 à 6, de préférence en une quantité sensoriellement efficace.

13. Produit parfumé selon la revendication 12, dans lequel ledit produit est choisi dans le groupe constitué par :
les extraits de parfum, les eaux de parfum, les eaux de toilette, les lotions après-rasage, les eaux de Cologne, les produits avant-rasage, les splash cologne, les lingettes parfumées rafraîchissantes, les produits de nettoyage acides, alcalins et neutres, les produits à rafraîchir les textiles, les aides à repasser, les lessives liquides, les lessives en poudre, les produits de traitement préalable du linge, les adoucissants de linge, les savons de lavage, les pastilles de lavage, les désinfectants, les désinfectants de surface, les produits à améliorer l'air, les aérosols, les cires et polis, les produits de toilette, les crèmes et lotions à mains, les crèmes et lotions à pieds, les crèmes et lotions à épiler, les crèmes et lotions après-rasage, les crèmes et lotions de bronzage, les produits pour les soins des cheveux, les produits désodorisants et antiperspirants, les produits de la cosmétique décorative, les bougies, les huiles pour lampes, les bâtons d'encens, les insecticides, les répulsifs et les carburants
et/ou
la part du mélange de substances odoriférantes selon l'une quelconque des revendications 1 à 6 dans le produit parfumé est comprise entre 0,01 et 10 % en poids, de préférence entre 0,1 et 5 % en poids et de manière particulièrement préférée entre 0,25 et 3 % en poids, respectivement par rapport à la masse totale du produit parfumé.

14. Procédé de préparation d'un produit parfumé, comprenant les étapes :
i) fournir un mélange de substances odoriférantes selon l'une quelconque des revendications 1 à 6 ou préparer un mélange de substances odoriférantes selon un procédé selon l'une quelconque des revendications 7 à 8,
ii) fournier les autres composants du produit parfumé et
iii) mettre en contact les autres composants du produit parfumé, fournis à l'étape ii), avec une quantité sensoriellement efficace du mélange de substances odoriférantes fourni à l'étape i) ; la quantité du composé de la formule (I) étant suffisante pour intensifier la fraîcheur naturelle et/ou le rayonnement d'une, de plusieurs ou de l'ensemble des substance(s) odoriférante(s) des composants (b) et/ou (c) et/ou pour masquer ou pour réduire des notes graisseuses, techniques et/ou métalliques
ou
I) fournir les composants du produit parfumé qui ne sont pas de composants (a), (b) ou bien (c) d'un mélange de substances odoriférantes tel que défini dans l'une quelconque des revendications 1 à 6
II) mélanger les composants du produit parfumé, fournis à l'étape I), aux composants (b) et/ou (c) d'un mélange de substances odoriférantes tel que défini dans l'une quelconque des revendications 1 à 6, de sorte qu'un mélange résulte dans lequel le ou les composant(s) (b) et/ou (c) sont présents en une quantité sensoriellement efficace,
III) mettre en contact le mélange préparé à l'étape II) avec une quantité du composé de la formule (I), la quantité du composé de la formule (I) étant suffisante pour intensifier la fraîcheur naturelle et/ou le rayonnement d'une, de plusieurs ou de l'ensemble des substance(s) odoriférante(s) des composants (b) et/ou (c) et/ou pour masquer ou pour réduire des notes graisseuses, techniques et/ou métalliques.

15. Utilisation du composé de la formule (I) 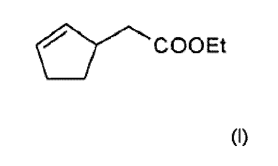 pour intensifier la fraîcheur naturelle et/ou le rayonnement et/ou pour masquer ou pour réduire des notes graisseuses, techniques et/ou métalliques d'une ou de plusieurs substance(s) odoriférante(s) différente(s) du composé de la formule (I), en particulier ayant une note olfactive fleurie, en particulier le jasmin,
de préférence,
la ou les substance(s) odoriférante(s) différente(s) du composé de la formule (I) étant choisie(s) dans les composants (b) et/ou (c) d'un mélange de substances odoriférantes selon l'une quelconque des revendications 1 à 6.
